# EUROPÄISCHE PATENTANMELDUNG

(11) **EP 4 792 838 A1**
(43) Veröffentlichungstag der Anmeldung: **19.08.2026**
(21) Anmeldenummer: 26158065.8
(22) Anmeldetag: 12.02.2026
(51) Int. Cl.: A61L 2/07

(54) **AUTOKLAV MIT EINER SICHERHEITSEINRICHTUNG EIN EINEM AUSBLASBEHÄLTER, REINRAUM UND VERFAHREN ZUM BETREIBEN DES AUTOKLAVS**

(30) Priorität: 14.02.2025 DE 102025105686
(71) Anmelder: Systec GmbH & Co. KG, 35440 Linden (DE)
(72) Erfinder: WEDEKING, Tim, 49479 Ibbenbüren (DE); SCHMIDT, Stephan, 49492 Westerkappeln (DE); JANNEK, Hubert, 97828 Marktheidenfield (DE); IHME, Andreas, 49525 Lengerich (DE); WINKELMANN, Jörg, 35435 Wettenberg (DE)
(74) Vertreter: Schulze Horn, Kathrin

(57) **Zusammenfassung**

1. Autoklav (100) zum Sterilisieren von Gegenständen, insbesondere von mit Mikroorganismen kontaminierten labortechnischen, medizinischen oder pharmazeutischen Geräten oder Produkten, mittels erhitztem, unter Druck stehendem Wasserdampf, wobei der Autoklav (100) mindestens einen druckfesten Autoklavbehälter (101) mit einer oder mehreren druckdicht verschließbaren Zugangsöffnungen, einen Dampfgenerator mit einer Heizeinrichtung und eine Sicherheitseinrichtung aufweist, wobei über eine Berstscheibe (104), ein Sicherheitsventil (105) und eine Ausblasleitung (102) der Autoklavbehälter (101) mit einem Ausblasbehälter (103) im S3/S4 Bereich verbunden ist,
**dadurch gekennzeichnet**,
dass der Autoklavbehälter (101) und der Ausblasbehälter (103) derart dimensioniert sind, dass der Ausblasbehälter (103) innerhalb des Autoklavbehälters (101) oder der Autoklavbehälter angeordnet ist.

## Beschreibung

Die vorliegende Erfindung betrifft einen Autoklav zum Sterilisieren von Gegenständen, insbesondere von mit Mikroorganismen kontaminierten labortechnischen, medizinischen oder pharmazeutischen Geräten oder Produkten, mittels erhitztem, unter Druck stehendem Wasserdampf, wobei der Autoklav mindestens einen druckfesten Autoklavbehälter mit einer oder mehreren druckdicht verschließbaren Zugangsöffnungen, einen über eine Dampfzuleitung mit dem Inneren des Autoklavbehälters verbundenen Dampfgenerator mit einer Heizeinrichtung und eine Sicherheitseinrichtung aufweist, wobei über eine Ausblasleitung eine Eingangsseite der Sicherheitseinrichtung mit dem Inneren des Autoklavbehälters und eine Ausgangsseite der Sicherheitseinrichtung mit einem Ausblaseingang des Ausblasbehälters verbunden ist. Außerdem betrifft die Erfindung einen Reinraum sowie ein Verfahren zum Betreiben des Autoklavs.

Die Druckschrift DE 20 2015 008 363 U1 beschreibt einen Autoklav der vorstehend genannten Art. Dieser Autoklav besitzt einen Ausblasbehälter als Druckentlastungs- und Entspannungsbehälter. Das Einleiten von ausblasendem Dampf in den Ausblasbehälter erfolgt mittels Tauchrohr in eine Flüssigkeit. Ein Schauglas kann als Füllstandkontrolle am Ausblasbehälter dienen. Eine Sterilisation des Ausblasbehälters muss im Havarie- oder Störfall mittels Wasserstoffperoxid-Begasung oder Formalin-Begasung erfolgen. Gleiches gilt für einen Reinraum, in dem der Autoklav angeordnet sein kann.

Insbesondere ist der Ausblasbehälter atmosphärisch offen ausgebildet, d. h. er weist eine Auslassöffnung oder eine Ableitung auf, die einen großen Durchmesser aufweist und nicht mit einem weiteren Sicherheitsventil oder dergleichen verschlossen ist.

Tritt also in dem eigentlichen Autoklavbehälter ein Störfall auf so wird der Autoklavbehälter durch seine Sicherheitseinrichtung druckentlastet. Der über die Ausblasleitung aus dem Autoklavbehälter abgeführte Dampf wird dem Ausblasbehälter zugeführt, wo üblicherweise durch die dort vorhandene Flüssigkeit der insbesondere kontaminierte Dampf kondensiert wird. Dadurch wird dessen Temperatur und das Volumen insgesamt reduziert, so dass auch durch die atmosphärisch offene Ausgestaltung des Ausblasbehälters eine mögliche Beschädigung der gesamten Vorrichtung wirkungsvoll vermieden ist.

Üblicherweise sind derartige Autoklave in einem Reinraum angeordnet. In dem Reinraum können beispielsweise labortechnische Untersuchungen und/oder Produktionsabläufe für unter anderem pharmazeutische Produkte, mit Bakterien oder Viren kontaminierte Gegenstände und dergleichen ausgeführt werden. Tritt ein Störfall im Autoklav auf muss der Reinraum mit allen enthaltenen Gegenständen beispielsweise mit Wasserstoffperoxid H₂O₂ desinfiziert werden, insbesondere auch die Ausblasleitung sowie der Ausblasbehälter.

Weiterhin hat es sich gezeigt, dass beim Ausblasen des heißen Dampfs aus dem Autoklav bei den laborüblichen Maßstäben meist nur eine Menge von beispielsweise 0,2 l oder 200 cm³ im Ausblasbehälter kondensiert wird.

Nachteilig bei diesem Gebrauchsmuster ist ferner, dass bei einer unkontrollierten Erhöhung erst nach dem Bruch der Berstscheibe und/oder Öffnung des SV ein Abschalten der Anlage erfolgt und der Ausblasbehälter deshalb entsprechend groß dimensioniert werden muss, und dass die Leitungen und der Ausblasbehälter mittels Wasserstoffperoxid Begasung (H2O2) oder Formalin Begasung sterilisiert werden müssen.

Weitere Veröffentlichungen zum Stand der Technik sind die technische Stellungnahme des Expertenkreis "Labortechnik" (ELATEC) des Ausschusses für Biologische Arbeitsstoffe (ABAS- www.baua.de/abas) vom 8.12.2016 zum Thema Ableitung eines Druckanstiegs innerhalb der Kammer eines Autoklaven über die Ausblaseleitung des Sicherheitsventils oder der Berstscheibe im Sicherheits- und Schutzstufenbereich 3-4 sowie die Biostoffverordnung Stand Juli 2013 (Best.-Nr.: A 227 bei Publikationsversand der Bundesregierung, Postfach 48 10 09, 18132 Rostock E-Mail: publikationen@bundesregierung.de Internet: http://www.bmas.de) und die Sicherheitsnorm EN 61508

Nachteilig ist ferner, dass im Falle eines unkontrollierten Ausblasens eines Autoklaven über ein Sicherheitsventil oder eine Berstscheibe der ggf. kontaminierte, weil noch nicht zu Ende autoklavierte Inhalt des Dampfsterilisierautoklaven, unkontrolliert in einen Raum austritt oder über ein Dach aus dem Gebäude in die Umwelt herausgeführt wird.

Der Ausschuss für Biologische Arbeitsstoffe (ABAS) berät seit 1995 das Bundesministerium für Arbeit und Soziales (BMAS) in Fragen des Arbeitsschutzes bei Tätigkeiten mit biologischen Arbeitsstoffen. Der Expertenkreis ELATEC dieses Ausschuss hat in einer technischen Stellungnahme deshalb empfohlen, die Ausgänge der Sicherheitsventile und/oder der Berstscheiben über einen Ausblasbehälter zu führen, in dem der übersättigte Dampf mit den ggf. nicht deaktivierten biologischen Stoffen in einem Ausblasbehälter durch ein Wasserbad geleitet und abgekühlt werden kann. Damit reduziert sich das Volumen und der Druck im Ausblasbehälter. DE 20 2015 008 363 U1 beschreibt einen Ausblasbehälter als Druckentlastungs-Entspannungsbehälter, gekennzeichnet durch: eine Druckstaffelung p1>p2>p3 zwischen Berstdruck der Berstscheibe oder Öffnungsdruck des Sicherheitsventils der Autoklavbehälter p1, dem Druck im Ausblasbehälter p2 und dem Berstdruck der Berstscheibe des Ausblasbehälters p3 und eine Einbringung in den Ausblasbehälter mittels Tauchrohr in eine Flüssigkeit und ein Schauglas als Füllstandskontrolle am Ausblasbehälter. Eine Sterilisation des Ausblasbehälters, die im Havariefall mittels Wasserstoffperoxid Begasung (H2O2) oder Formalin Begasung erfolgen muss.

Zwei Ausführungen eines Ausblasbehälters nach dieser Bauart sind bekannt aus dem Paul-Ehrlich Institut in Langen, DE. Beiden Ausführungen ist gemeinsam, dass es sich um einen festverrohrten, festinstallierten Druckbehälter handelt. Eine Integration eines Ausblasbehälters in dieser Form ist mit vergleichsweise hohen Kosten verbunden, die sogar die Investition eines Autoklaven übersteigen können.

Nachteilig bei diesem Stand der Technik ist, dass üblicherweise der Autoklav beziehungsweise dessen Ausblasbehälter aufwändig abgebaut und separat desinfiziert werden muss. Hierzu sind meist mehrere Arbeitsschritte erforderlich und gegebenenfalls muss der Ausblasbehälter demontiert und separat aus einem Reinraum entfernt und desinfiziert werden.

Es stellt sich daher die Aufgabe, einen Autoklav und ein Verfahren zu dessen Betreiben sowie einen entsprechend ausgestatteten Reinraum anzugeben, so dass Gefahren für Mensch und Umwelt vermieden oder wenigstens vermindert werden.

Die Lösung der Aufgabe wird mit einem Autoklav gemäß des Patentanspruchs 1 erreicht. Weiterhin wird ein entsprechend ausgestatteter Reinraum angegeben sowie ein Verfahren zur Handhabung des erfindungsgemäßen Autoklavs.

Es wird eine Vorrichtung beansprucht mit folgenden möglichen Merkmalen, als mehrstufige Sicherheitseinrichtung für einen Autoklaven bestehend aus einem Druckschalter, einer Berstscheibe und einem Sicherheitsventil, um eine Reduzierung der Ausblasmenge in einen Ausblasbehälter bei einer eventuellen Öffnung des Sicherheitsventils zu erreichen, wobei der Druckschalter bereits deutlich unter dem Berstdruck der Berstscheibe die Anlage so schaltet, dass die Ventile zwischen Dampfgenerator und Autoklavbehälter sofort geschlossen und damit die Dampfzufuhr in den Behälter unterbrochen wird, wobei die Ventile der Druckluftzuführung zum Autoklavbehälter geschlossen und die Druckluftzufuhr in den Behälter unterbrochen wird, wobei die Stromversorgung des Dampfgenerator sicher unterbrochen wird, womit es zu keiner weiteren Dampferzeugung im Dampfgenerator kommen kann, die als kompakte abnehmbare Berstscheibe und / oder kompakt abnehmbares Sicherheitsventil hinter einer Berstscheibe, wobei das Sicherheitsventil oder die Berstscheibe in einem Autoklaven autoklaviert werden können oder als kompakter mobiler Ausblasbehälter hinter dem Sicherheitsventil eines Autoklaven, wobei der Ausblasbehälter in einem Autoklaven autoklaviert werden kann, wobei die zuführenden Leitungen zum Ausblasbehälter abgenommen und entweder in einem Autoklaven sterilisiert oder entsorgt werden können und wobei die Leitungen nach einem Ausblasen durch Sattdampf in einem speziellen Programmlauf des Autoklaven ebenfalls einfach sterilisiert werden können, oder auch als kompakter mobiler Ausblasbehälter hinter dem Sicherheitsventil eines Autoklaven, wobei bei Öffnung des Sicherheitsventils das Abgas durch eine Flüssigkeit im Ausblasbehälter geführt und abgekühlt wird, wodurch der Druck im Ausblasbehälter nahe dem Umgebungsdruck ist, ausgeführt ist.

Ferner wird ergänzend ein Verfahren mit den folgenden möglichen Merkmalen beansprucht, in welchem zusätzlich zu der SPS-seitigen Abschaltung eine hardwareseitige Abschaltung durch entweder eine sichere Unterbrechung der Stromzufuhr (Schütz mit Sicherheitsintegritätslevel 3 bzw. 4) sofort bei Erreichen des Ansprechdruckes des Druckschalters und eine sofortige und sichere Unterbrechung der Dampfzufuhr (Schütz mit Sicherheitsintegritätslevel 3 bzw. 4) durch in Reihe geschaltete normal closed Ventile zwischen Dampfgenerator und Autoklaven Kammer, die schließen, sobald die Stromzufuhr unterbrochen ist, oder in welchem im Havariefall die Ventile zum Autoklavbehälter geschlossen werden, die Berstscheibe, das Sicherheitsventil, und der Schlauch entnommen und diese mitsamt dem Ausblasbehälter in einem Autoklaven sterilisiert werden.

Zur Veranschaulichung der Erfindung erfolgt zunächst eine Beschreibung des Dampfsterilisierprozesses und der daran beteiligten Komponenten mit einem Autoklaven und einen Autoklavierprozess. In diesem Zusammenhang zu erwähnenden Komponenten sind: Autoklavbehälter, Dampfgenerator, Drucküberwachung, Steuerung, Abluftfilter, Sicherheitsventil, Berstscheibe, Bioshield, Ausblasebehälter und Sicherheitsbereiche S2, S3 und S4 nach Biostoffverordnung.

Es muss vermieden werden, dass im Falle eines unkontrollierten Ausblasens eines Autoklaven über ein Sicherheitsventil oder eine Berstscheibe der ggf. kontaminierte, weil noch nicht zu Ende autoklavierte Inhalt des Dampfsterilisierautoklaven, unkontrolliert in das Labor austritt oder über ein Dach aus dem Gebäude in die Umwelt herausgeführt wird.

Die Menge des ausgeblasenen Gases orientiert sich dabei an der Spezifikation des Sicherheitsventils und/oder der Berstscheibe des Autoklaven.

Ist der Berstdruck der Berstscheibe oder der Öffnungsdruck des Sicherheitsventils erreicht, wird solange ausgeblasen, bis im Falle einer Berstscheibe kein Überdruck mehr im Behälter vorhanden ist oder im Fall des Sicherheitsventils der Ansprechdruck des Sicherheitsventils unterschritten wird.

Es stellt sich also die Aufgabe eine Vorrichtung und ein Verfahren vorzustellen, mittels dessen
- bereits eine sichere Abschaltung der energiezuführenden Medien in den Autoklavbehälter erreichbar ist, bevor es zu einem Bersten der Berstscheibe und einem Öffnen des Sicherheitsventils kommt, und damit ein ausblasen zu vermeiden,
- den Autoklaven immer mit Berstscheibe und Sicherheitsventil auszustatten, damit es sicher nicht zu einem Schnüffeln des Sicherheitsventils kommen kann und nicht der ganze Inhalt des Autoklaven im Havariefall den Behälter verlässt, sondern durch ein Schließen des Sicherheitsventils im Autoklaven belassen wird und
- damit einen kleinen, mobilen Ausblasbehälter zu nutzen, der im Falle einer Kontamination mit biologischen Stoffen einfach im Autoklaven sterilisiert werden kann. Der Ausblasbehälter ist im Ausblasfall einfach zusammen mit Berstscheibe und Sicherheitsventil sowie Ausblaseleitung entnehmbar und separat sterilisierbar. Das ist für Labore ein Handlingsvorteil gegenüber einem festverbauten Ausblasbehälter, außerdem benötigt er im Labor wenig Platz und
- um eine einfache Sterilisation und Wartung des Behälters und der zu- und abführenden Leitungen zu ermöglichen und eine Begasung beispielsweise mit Formalin oder H2O2 zu vermeiden und
- um Kosten bei der Installation und insbesondere in einem Havariefall zu senken und Umwelt- und Personengefährdungen einer Begasung zu vermeiden. Die Rückführung im Überdruckfall erfolgt in den S3 Bereich, der laut Biostoffverordnung bereits so gesichert sein muss, dass es nicht zu einem Austritt kommen kann für "...Biostoffe, die eine schwere Krankheit beim Menschen hervorrufen und eine ernste Gefahr für Beschäftigte darstellen können..."

Der Autoklav gemäß Erfindung ist derart ausgestaltet, dass der Ausblasbehälter relativ zum eigentlichen Autoklavbehälter derart dimensioniert ist, dass der Ausblasbehälter im angeschlossenen Autoklavbehälter oder einem anderen im Labor befindlichen Autoklavbehälter einbringbar oder anzuordnen ist. Somit kann nach Auftreten eines Störfalls im Autoklav, der durch die Sicherheitseinrichtung zum Ausblasen des insbesondere kontaminierten heißen Dampfs im Autoklavbehälter über die Ausblasleitung in den Ausblasbehälter führt, dieser Ausblasbehälter unmittelbar durch eine Person demontiert und im Autoklavbehälter beziehungsweise in einem Autoklavbehälter gleicher oder größerer Bauart sterilisiert werden kann. Insbesondere sind der oder die mehreren baugleichen Autoklave in einem Reinraum angeordnet.

Hierzu muss lediglich eine Person in einem entsprechenden Schutzanzug durch eine Sicherheitsschleuse den Reinraum betreten, den kontaminierten Ausblasbehälter demontieren und im Autoklavbehälter anordnen und sterilisieren.

Es versteht sich, dass simultan oder zeitlich versetzt der Reinraum selbst mit beispielsweise Wasserstoffperoxid oder sonstigen bekannten Desinfektionsmitteln gereinigt wird.

Weiterhin ist es ersichtlich, dass der Autoklav in an sich bekannter Weise mit einem Dampfgenerator zur Heißdampferzeugung, einer Sicherheitseinrichtung zur Vermeidung von Beschädigungen im Störfall sowie einer Steuerung ausgestattet ist. Hierzu kann der Autoklav beispielsweise entsprechend der DE 10 2020 121 083 A1 ausgebildet und mit einem Ausblasbehälter beispielsweise entsprechend der DE 20 2015 008 363 U1 ausgestattet sein.

Der Vorteil der Erfindung besteht darin, dass ein kontaminierter Ausblasbehälter unmittelbar beispielsweise im Reinraum sterilisiert werden kann. Er muss nicht aufwändig separat deinstalliert und beispielsweise aus dem Reinraum entfernt werden. Dies kann auch durch das Laborpersonal selbst erfolgen.

Bevorzugte Weiterbildungen und Ausgestaltungen des Autoklavs sind in den Unteransprüchen angegeben.

Bevorzugt ist auch die Ausblasleitung, die entsprechend dimensioniert ist, zusätzlich im Autoklavbehälter einbringbar oder anzuordnen.

Bevorzugt ist eine abnehmbare Berstscheibe und / oder ein kompakt abnehmbares Sicherheitsventil hinter einer Berstscheibe zusätzlich im Autoklavbehälter einbringbar oder anzuordnen.

In vorteilhafter Weise ist die Ausblasleitung flexibel ausgebildet, um platzsparend im Autoklavbehälter angeordnet zu werden.

Weiterhin ist vorgeschlagen, dass der Ausblasbehälter mit einer Flüssigkeit zumindest teilweise befüllt ist, um den hindurchströmenden Heißdampf aus dem Autoklavbehälter abzukühlen und zu kondensieren. Hierdurch wird das Volumen erheblich verringert und die Temperatur reduziert.

Schließlich kann dem Ausblasbehälter ein zusätzlicher Kondensator vorgeschaltet werden. Der vorzugsweise ebenfalls mit Wasser befüllte Kondensator reduziert das Volumen und die Temperatur des durchströmenden üblicherweise heißen und gasförmigen Mediums zusätzlich, so dass keine unerwünschten Überdrücke auftreten können.

Weiterhin wird vorgeschlagen, dass ein oder mehrere Autoklave in einem Reinraum angeordnet sind. In diesem Reinraum können unter anderem Untersuchungen an kontaminierten Proben und/oder Produktionsprozesse beispielsweise für giftige oder infektiöse Produkte durchgeführt werden. Dabei ist der Reinraum beispielsweise mit Schleusen zum Betreten sowie den vorgeschriebenen Sicherheitseinrichtungen ausgestattet, um ein Austreten von insbesondere biologisch gefährlichen Schadstoffen zu vermeiden.

Im nachfolgenden Teil der Beschreibung werden weitere Ausführungen zum Aufbau des Autoklaven gemacht sowie Hinweise zu dessen Handhabung gegeben.

Für die sichere Ausblaseinrichtung für Autoklaven in den Raum mit dem höheren Sicherheitslevel (Reinraum) ist vorzugsweise vorgesehen, dass entweder diese eine mehrstufige Sicherheitseinrichtung für einen Autoklaven bestehend aus einem Druckschalter, einer Berstscheibe und einem Sicherheitsventil umfasst, um eine Reduzierung der Ausblasmenge in einen Ausblasbehälter bei einer eventuellen Öffnung des Sicherheitsventils zu erreichen, wobei der Druckschalter mit hohem Sicherheitsintegritätslevel (SIL 3 bzw. SIL 4) bereits deutlich unter dem Berstdruck der Berstscheibe die energieeinbringenden Medien unterbricht sowie den Dampfgenerator stromlos schaltet, wobei die normal closed Ventile zwischen Dampfgenerator und Autoklavbehälter sofort geschlossen und damit die Dampfzufuhr in den Behälter unterbrochen wird, oder
ein kompakter mobiler im Reinraum angeordneter Ausblasbehälter für einen Autoklaven, wobei das Sicherheitsventil im Reinraum angeordnet ist, wobei dem Sicherheitsventil eine Berstscheibe im Reinraum vorgeordnet werden kann, wobei die Abführleitung von dem Sicherheitsventil in einen Ausblasbehälter innerhalb des Reinraums geführt wird, wobei diesem Ausblasbehälter ein Kondensator vorgeschaltet werden kann, wobei der Ausblasbehälter mit Wasser zum Niederschlag des Dampfes befüllt werden kann, wobei der Ausblasbehälter abnehmbar aufgebaut wird und wobei der Ausblasbehälter in einem Autoklaven sterilisiert werden kann, wobei die zuführenden Leitungen zum Ausblasbehälter abgenommen und entweder in einem Autoklaven sterilisiert oder entsorgt werden können und wobei die Berstscheibe und das Sicherheitsventil abgenommen und in einem Autoklaven sterilisiert oder entsorgt werden können.

Dabei ist unter dem Begriff "diesem Ausblasebehälter ein Kondensator vorgeschaltet" zu verstehen, dass bei bestimmungsgemäßem Gebrauch der gesamten Vorrichtung in einer Strömungsrichtung der die Vorrichtung durchströmenden Medien gesehen vor dem Ausblasbehälter ein zusätzlicher Kondensator angeordnet sein kann. Dieser ist vorzugsweise ebenfalls mit Wasser gefüllt, um das durchströmende Medium abzukühlen und in seinem Volumen zu reduzieren. Dabei versteht es sich, dass der Kondensator und der Autoklavbehälter ebenfalls derart dimensioniert sind, dass der Kondensator ebenfalls in den Autoklavbehälter oder einen weiteren Autoklavbehälter eingebracht und darin sterilisiert werden kann, ohne also einen Reinraum zu verlassen.

Die Aufgabe wird durch die Merkmale des Patentanspruchs 1 gelöst. Weitere Ausführungsformen sind durch die Merkmale der Unteransprüche vorgesehen.

Weitere Einzelheiten und Vorteile der Erfindung werden anhand der beigefügten Zeichnungen beschrieben.
- Fig 1:: ein Ausblasbehälter innerhalb des Sicherheitsbereichs angeordnet
- Fig 2:: ein Ausblasbehälter mit-vorgeschaltetem zusätzlichem Kondensator innerhalb des Sicherheitsbereichs
- Fig 3:: schematischer Aufbau

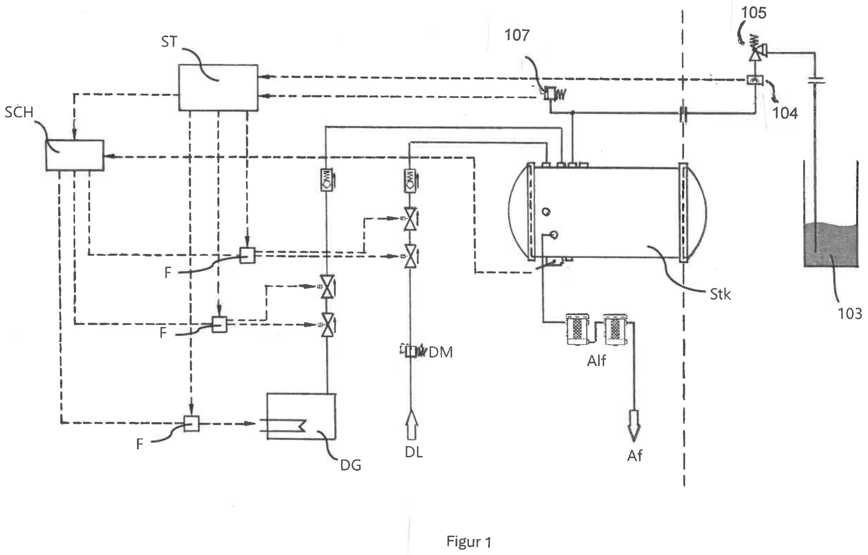

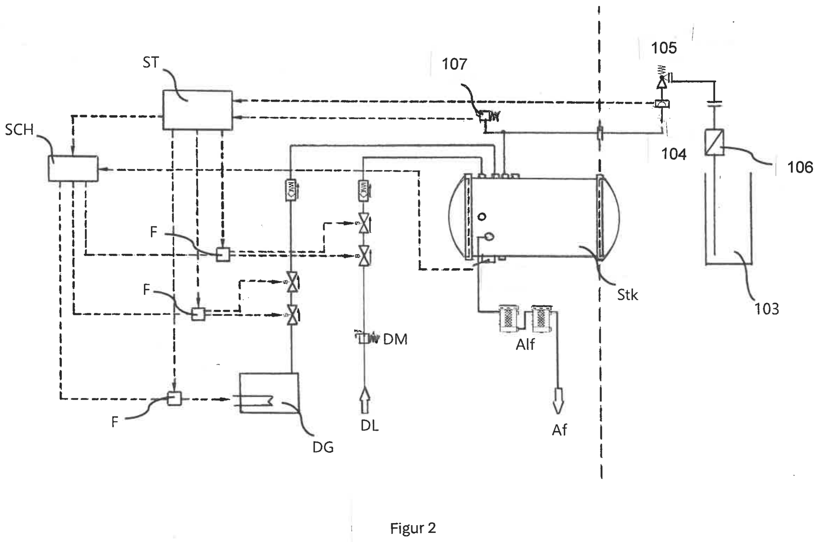

Fig. 1 und 2 zeigen das R&I Bild der erfindungsgemäßen Ausblaseinrichtung mit einem autoklavierbaren Ausblasbehälter und einer mehrstufigen Sicherheitseinrichtung:
1. Die mehrstufige Sicherheitseinrichtung besteht aus einem Druckschalter (107), einer Berstscheibe (104) am Autoklavbehälter (101), einem Sicherheitsventil (105), einer Ausblaseleitung (102), einem Kondensator (106) und einem Ausblasebehälter (103). Diese Sicherheitseinrichtung ist im Sicherheitsbereich des Labors (S1-S4) platziert.
2. Der Druckschalter (107 ) wird sicher ausgeführt (beispielsweise eine Ausführung mit hohem Sicherheitsintegritätslevel). Der Druckschalter (107) ist auf einen festen Wert p1 eingestellt. Bei Erreichen des Wertes werden die einergieeinbringenden Medien sicher unterbrochen, beispielsweise über ein Schütz mit hohem Sicherheitsintegritätslevel. Damit wird das Ventil zwischen Dampfgenerator und Autoklavbehälter (101) geschlossen und die Dampfzufuhr in den Behälter (101) sicher unterbrochen. Außerdem wird das Ventil der Druckluftzuführung geschlossen und die Druckluftzufuhr in den Behälter (101) sicher unterbrochen. Außerdem wird die Stromversorgung des Dampfgenerators sicher unterbrochen, womit es zu keiner weiteren Dampferzeugung im Dampfgenerator kommen kann.
3. Bei Bersten der Berstscheibe (104) p2 (wobei gilt p2>p1) werden die einergieeinbringenden Medien sicher unterbrochen, beispielsweise über ein Schütz mit hohem Sicherheitsintegritätslevel. Damit wird das Ventil zwischen Dampfgenerator und Autoklavbehälter (101) geschlossen und die Dampfzufuhr in den Behälter (101) sicher unterbrochen. Außerdem wird das Ventil der Druckluftzuführung geschlossen und die Druckluftzufuhr in den Behälter (101) sicher unterbrochen. Außerdem wird die Stromversorgung des Dampfgenerators sicher unterbrochen, womit es zu keiner weiteren Dampferzeugung im Dampfgenerator kommen kann.
4. Hinter dem Autoklavbehälter (101) wird eine trennbare Vorrichtung, beispielsweise ein Triclamp, ausgeführt. An der trennbaren Vorrichtung wird eine Berstscheibe (104) angebracht.
5. Hinter der Berstscheibe (104) wird eine trennbare Vorrichtung, beispielsweise ein Triclamp, ausgeführt. An der trennbaren Vorrichtung wird ein Sicherheitsventil (105) angebracht.
6. Hinter dem Sicherheitsventil (105) wird eine trennbare Vorrichtung, beispielsweise ein Triclamp, ausgeführt. An der trennbaren Vorrichtung wird eine druckfeste flexible Verbindung, beispielsweise ein druckfester Schlauch zum Ausblasbehälter (103) geführt. Im Havariefall kann die flexible Verbindung entnommen und mitsamt dem Ausblasbehälter (103) im Autoklaven (100) sterilisiert werden.
7. Der Ausblasbehälter (103) wird in den Bereich mit dem höheren Sicherheitslevel (z.B. S3/S4) gestellt und ist offen zum S3/S4 Bereich. Somit entspricht der Druck p4 im Ausblasbehälter (103) dem Druck im S3/S4 Bereich und ist damit deutlich geringer als der Druck p3 am Sicherheitsventil (105) des Autoklaven (100).

Im Folgenden werden Ausführungsbeispiele der Erfindung anhand von Zeichnungen erläutert.

Der Autoklav 100 umfasst einen druckfesten Autoklavbehälter 101 mit einem Inneren zur Aufnahme von zu sterilisierenden Gegenständen oder Produkten.

Zum Einbringen und Entnehmen der Gegenstände weist der Autoklavbehälter 101 wenigstens eine hier nicht näher dargestellte, druckdicht verschließbare Zugangsöffnung auf. Weiterhin umfasst der Autoklav 100 in an sich bekannter Weise weitere hier dargestellte Komponenten wie einen Dampfgenerator zur Zuführung von heißem Wasserdampf, eine vorzugsweise mehrstufige Sicherheitseinrichtung wie Berstscheiben 104, Überdruck- und/oder Rückschlagventile, Ableitungen und dergleichen zur Vermeidung von Beschädigungen im Falle eines Störfalls sowie einen Ausblasbehälter 103, der gegebenenfalls mit einer Flüssigkeit zum Kondensieren des Abdampfs zumindest teilweise befüllt sein kann. Im Falle eines Störfalls kann gesteuert durch ein Sicherheitsventil 105, eine Berstscheibe 104 oder dergleichen über eine vorzugsweise flexible Ausblasleitung 102, der Heißdampf aus dem Autoklavbehälter 101 in den Ausblasbehälter 103 geführt werden. Der Ausblasbehälter 103 atmosphärisch offen, so dass sich im Ausblasbehälter 103 kein Überdruck aufbauen kann.

Insbesondere sind ein oder mehrere baugleiche Autoklave 100 in einem Reinraum angeordnet.

Tritt ein Störfall auf, so sind der Autoklav 100 insgesamt beispielsweise mit schädlichen Mikroorganismen sowie der umgebende Reinraum kontaminiert.

In diesem Fall kann eine Person im Schutzanzug den Reinraum durch eine entsprechende Schleuse betreten. Sie kann dann den Ausblasbehälter 103 sowie gegebenenfalls die Ausblasleitung 102, das Sicherheitsventil 105, die Berstscheibe 104 über an sich bekannte Triclamps demontieren und im Autoklavbehälter 101, vorzugsweise eines benachbart angeordneten baugleichen Autoklav 100, einbringen und dort sterilisieren. Ebenso kann der Reinraum selbst in an sich bekannter Weise mit Wasserstoffperoxid desinfiziert werden.

Somit kann die gesamte Anlage schnell und ohne erheblichen Aufwand wieder in betriebsfähigen Zustand versetzt werden.

Hierzu sind der Autoklavbehälter 101 und der Ausblasbehälter 103 derart dimensioniert, dass der Ausblasbehälter 103 sowie gegebenenfalls die Ausblasleitung 102 sowie Berstscheibe 104 und Sicherheitsventil 105 innerhalb des Autoklavbehälters 101 anzuordnen sind. Das bedeutet, dass der Ausblasbehälter 103 weniger voluminös oder kleiner ist als der Autoklavbehälter 101.

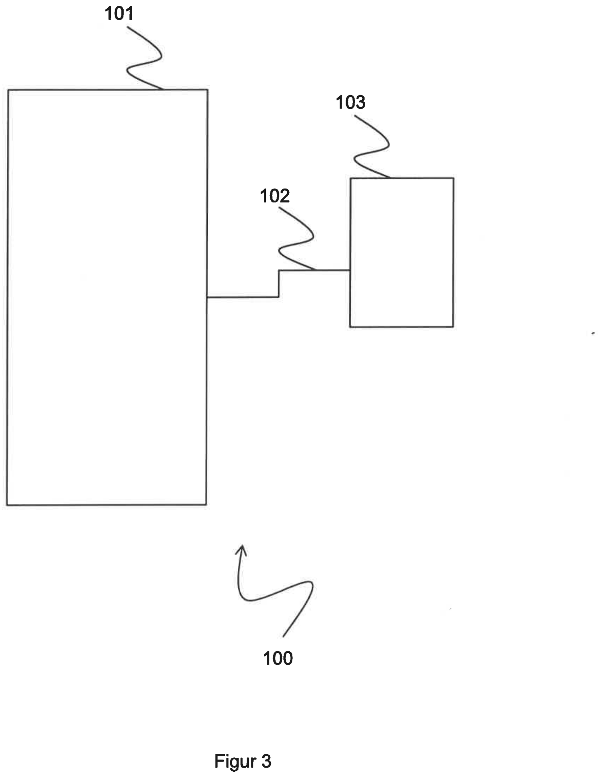

Sämtliche Komponenten des Autoklav 100 können entsprechend dem Stand der Technik wie vorstehend angegeben ausgebildet sein. Weiterhin kann, wie in Figur 3 verdeutlicht, dem Ausblasbehälter 103 ein zusätzlicher Kondensator 106 vorgeschaltet sein, der vorzugsweise ebenfalls mit Wasser zum Kondensieren des Mediums gefüllt ist und der gemeinsam mit dem Ausblasbehälter 103 im Autoklavbehälter 101 zur Reinigung anzuordnen ist.

In den Figuren 1und 2 sind zwei Versionen der gesamten Vorrichtung abgebildet, in Figur 1 ist der Ausblasbehälter 103 innerhalb des Sicherheitsbereichs angeordnet und in Figur 2 ist ein zusätzlicher Kondensator 106 innerhalb des Sicherheitsbereichs vorgeschaltet.

Schließlich ist in Figur 3 ein grundsätzlicher Aufbau der gesamten Vorrichtung schematisch wiedergegeben.

Es versteht sich, dass in allen Figuren die jeweils gleichen Bezugszeichen beziehungsweise Abkürzungen auch die gleichen Komponenten bezeichnen. Weiterhin ist ersichtlich, dass alle Komponenten, die mit gestrichelten oder durchgehenden Linien miteinander verbunden sind, jeweils miteinander in Wirkverbindung stehen, und dass die Pfeile an den Linien jeweils die bestimmungsgemäße Strömungsrichtung eines Fluids durch die gesamte Vorrichtung anzeigen.

### Bezugszeichenliste:

### Zeichen Bezeichnung

- 100: Autoklav
- 101: Autoklavbehälter
- 102: Ausblasleitung
- 103: Ausblasbehälter
- 104: Berstscheibe
- 105: Sicherheitsventil
- 106: Kondensator
- 107: Druckschalter

### Abkürzungen

- DG: Dampfgenerator
- RV: Rückschlagventil
- SPS: Speicherprogrammierbare Steuerung
- P: Druck
- p1: Druck am Druckschalter
- p2: Druck an der Berstscheibe
- p3: Druck am Sicherheitsventil des Autoklaven
- p4: Druck im Ausblasbehälter
- Sch: Schaltung
- St: Steuerung
- F: Freigabe
- Stk: Sterilisierkammer
- Alf: Abluftfilter
- Af: Abfluss
- DI: Druckluft
- Dm: Druckminderer

## Patentansprüche

1. Autoklav (100) zum Sterilisieren von Gegenständen, insbesondere von mit Mikroorganismen kontaminierten labortechnischen, medizinischen oder pharmazeutischen Geräten oder Produkten, mittels erhitztem, unter Druck stehendem Wasserdampf, wobei der Autoklav (100) mindestens einen druckfesten Autoklavbehälter (101) mit einer oder mehreren druckdicht verschließbaren Zugangsöffnungen, einen Dampfgenerator mit einer Heizeinrichtung und eine Sicherheitseinrichtung aufweist, wobei über eine Berstscheibe (104), ein Sicherheitsventil (105) und eine Ausblasleitung (102) der Autoklavbehälter (101) mit einem Ausblasbehälter (103) im S3/S4 Bereich verbunden ist,
**dadurch gekennzeichnet,**
**dass** der Autoklavbehälter (101) und der Ausblasbehälter (103) derart dimensioniert sind, dass der Ausblasbehälter (103) innerhalb des Autoklavbehälters (101) oder der Autoklavbehälter angeordnet ist.

2. Autoklav (100) nach Anspruch 1, **dadurch gekennzeichnet, dass** auch die Ausblasleitung (102) zusätzlich innerhalb des Autoklavbehälters (101) anzuordnen ist.

3. Autoklav (100) nach Anspruch 1, **dadurch gekennzeichnet, dass** eine Berstscheibe (104) und ein Sicherheitsventil (105) so enthalten und angeordnet sind, dass Berstscheiben (104) und Sicherheitsventil (105) zusätzlich innerhalb des Autoklavbehälters (101) anzuordnen sind.

4. Autoklav (100) nach einem der Ansprüche 1 bis 3, **dadurch gekennzeichnet, dass** die Ausblasleitung (102) flexibel ausgebildet ist.

5. Autoklav (100) nach einem der Ansprüche 1 bis 4, **dadurch gekennzeichnet, dass** im Ausblasbehälter (103) eine Flüssigkeit zum Kondensieren vorgesehen ist.

6. Autoklav (100) nach einem der Ansprüche 1 bis 5 **dadurch gekennzeichnet, dass** dem Ausblasbehälter (103) ein Kondensator (106) vorgeschaltet ist.

7. Autoklav (100) nach einem der Ansprüche 1 bis 6 **dadurch gekennzeichnet, dass** ein Druckschalter (107) als Sicherheitseinrichtung vorgesehen ist.

8. Reinraum umfassend einen Autoklav (100) nach einem der Ansprüche 1 bis 7.

9. Verfahren zum Betreiben eines Autoklavs (100) nach einem der Patentansprüche 1 bis 7,
**dadurch gekennzeichnet,**
**dass** im Falle eines Störfalls der Ausblasbehälter (103) in den Autoklavebehälter (101) und / oder in mindestens einem weiteren Autoklavbehälter zum Sterilisieren eingebracht wird.

10. Verfahren nach Anspruch 1 - 7, **dadurch gekennzeichnet, dass** auch die Ausblasleitung (102) in den Autoklavbehälter (101) und / oder in den weiteren Autoklavbehältern zum Sterilisieren eingebracht wird.

11. Verfahren nach Anspruch 9 oder 10, **dadurch gekennzeichnet, dass** ein den Ausblasebehälter (103) vorgeschalteter Kondensator (106) in den Autoklavbehälter (101) und / oder den weiteren Autoklavbehältern zum Sterilisieren eingebracht wird.

12. Verfahren nach Anspruch 9 bis 11, **dadurch gekennzeichnet, dass** die Berstscheibe (104) und das Sicherheitsventil (105) in einen Autoklavbehälter (101) und / oder den weiteren Autoklavbehältern zum Sterilisieren eingebracht wird.
